# EUROPEAN PATENT SPECIFICATION

(11) **EP 2 547 964 B1**
(45) Date of publication and mention of the grant of the patent: **13.04.2016**
(21) Application number: 10813115.2
(22) Date of filing: 30.12.2010
(51) Int. Cl.: F24F 3/16, A61G 10/02, A61M 16/00, A61G 13/00

(54) **TREATMENT OF ASTHMA, ALLERGIC RHINITIS AND IMPROVEMENT OF QUALITY OF SLEEP BY TEMPERATURE CONTROLLED LAMINAR AIRFLOW TREATMENT**
BEHANDLUNG VON ASTHMA, ALLERGISCHER RHINITIS UND VERBESSERUNG DER SCHLAFQUALITÄT DURCH TEMPERATURGESTEUERTE LAMINARE LUFTSTROMBEHANDLUNG
TRAITEMENT DE L' ASTHME, DES RHUMES ALLERGIQUES ET AMELIORATION DE LA QUALITE DU SOMMEIL PAR TRAITEMENT DE FLUX D'AIR LAMINAIRE THERMOCOMMANDE

(30) Priority: 08.06.2010 EP 10005880; 08.06.2010 US 352517 P; 04.05.2010 EP 10004690; 04.05.2010 US 331080 P; 16.03.2010 EP 10002740; 16.03.2010 US 314345 P
(43) Date of publication of application: 23.01.2013
(60) Divisional application: 13168525.7
(73) Proprietor: Airsonett AB, 262 72 Ängelholm (SE)
(72) Inventor: KRISTENSSON, Dan, Allan, Robert, SE-26263 Ängelholm (SE); SVENSSON, Pal, Martin, SE-30244 Halmstad (SE); KORNFELD, Mark, SE-22649 Lund (SE)
(74) Representative: Budde Schou A/S
(86) International application number: PCT/IB2010/003369
(87) International publication number: WO 2011/114186

(56) References cited:
- WO-A1-01/94853
- WO-A1-2004/092658
- WO-A1-2007/100286
- WO-A1-2008/136740

## Description

### Field of the invention

It has been found that the relative particle and allergen concentration in air during situations of or corresponding to sleep is generally higher than in other situations and elsewhere in normal bed- or living-rooms due to body convection. Human body generated convection currents passing close by the breathing zone enforce and condense emissions from the all important reservoirs in the beddings distorted due to movements in the bed.

This invention relates in general to devices for displacing body convection and thereby reducing exposure to allergens and other airborne fine particles within a personal breathing zone during situations of or corresponding to sleep thereby reducing or removing symptoms of asthma and allergic rhinitis while improving quality of sleep and in particular to devices that utilize Temperature controlled Laminar Airflow (abbreviated TLA from herein and onwards).

### Background

With an estimated 300 million affected individuals worldwide asthma is considered a global problem. The World Health Organization has estimated that 15 million disability-adjusted life years are lost annually due to asthma (representing 1% of the total global disease burden). In terms of mortality it has been estimated that asthma claims 250,000 deaths per year (see ref. 1).

The monetary costs including those of health care systems and indirect costs such as time lost from work and premature death are substantial (see ref. 2). The costs involved in treatment are high, however, the costs of not treating asthma are considered even higher (see ref. 3).

Asthma is known to be an inflammatory disease in terms of pathology involving many cell types and cellular elements which in turn causes inflammation and airway hyperresponsiveness in a not well-understood fashion. The key cellular mediators of asthma are believed to be chemokines, cysteinyl leukotrienes, cytokines, histamine, prostaglandin D2 and nitric oxide (NO).

The main physiological symptoms of asthma are episodic airway obstruction causing expiratory airflow limitation breathlessness, wheezing, cough and chest tightness (see ref. 4).

The specific definition of asthma includes two domains (symptoms and variable airway obstruction) and additional two domains (airway inflammation and airway hyperresponsiveness (AHR)), wherein the latter domains are thought to characterize the underlying disease process (see ref. 5).

Certain host factors usually cause the development of asthma whereas environmental factors typically trigger asthma symptoms (although some factors do both). Host factors include sex, obesity and genetic background whereas environmental factors include infection and tobacco smoke and specifically allergens originating from e.g. furred animals, domestic mites, fungi, molds and yeast (see ref. 6). Exercise, especially in dry cold air, can also trigger asthma (see ref. 7).

Efforts have been made by different academic societies to standardize the terms such as asthma severity and control. Astma control refers to the extent to which the symptoms of asthma is reduced or removed by treatment. The degree of severity is based on the intensity of treatment required to achieve good asthma control.

Asthma may be clinically diagnosed based on a number of physiological parameters, including episodic breathlessness, wheezing, cough and chest tightness (see ref. 4). However, the heterogeneity of symptoms (or phenotypes) makes it less easy to diagnose asthma.

Common methods of assessing and monitoring asthma in terms of airflow limitation in patients over 5 years of age include forced expiratory volume in the first second (FEV₁) and peak expiratory flow (PEF) measurements.

The level of exhaled NO is a non-invasive marker of airway inflammation in asthma patients since elevated levels of fractional exhaled nitric oxide (FENO) is often elevated in such patients. Therefore monitoring FENO levels may provide an indication of the severity of asthma. It is shown that FENO is clearly glucocorticosteroid dependent but patients with poor asthma control may still have elevated FENO levels in spite of glucocorticosteroid medication (see ref. 8).

Measurements on lung function are useful for evaluating the severity of asthma as well as effects of treatment and can with advantage be supplemented with quality of life scores (see ref. 9).

Thus, other methods for monitoring the severity of asthma include the use of questionnaires, such as the mini asthma quality of life questionnaires (mini-AQLQ). The mini-AQLQ provides a score based on 15 questions in the areas of symptoms, activity limitation, emotional function and environmental stimuli. The questionnaire is considered a simple and robust tool for research and quality of care monitoring in primary care at the group level. The questionnaire can also be used with confidence in large clinical trials and surveys (see refs. 9-11).

A similar pediatric asthma quality of life questionnaire (PAQLQ) exists for use in the pediatric population (see refs. 12-13).

A number of treatments for achieving asthma control are available to the patient including medicaments such as inhaled corticosteroids (ICS) including budesonide and fluticasone, short-acting β₂ agonists (SABA), long-acting β₂ agonists (LABA), leukotriene modifiers, sustained release theophylline and anti IgE-treatment.

Furthermore non-invasive measures including the removal of triggering factors are also possible.

In allergic asthma the inflammatory disease is mainly driven by exposure to inhalant allergens to which the patient is sensitised. Minimising the impact of inhalant allergens such as those from house dust mites, cats, and dogs is a first step in reducing the severity of asthma (see ref. 14). Experiences from high altitude studies suggest that long term allergen avoidance leads to a decrease in airway inflammation with consequent symptom improvement. The studies also suggest that it is essential to achieve and maintain a major reduction in allergen levels (see refs. 15-16).

Allergic rhinitis and asthma are mediated by similar allergic mechanisms and they may represent two manifestations of the same united airway disease.

Allergic rhinitis is a collection of symptoms such as watery nasal discharge, nasal congestion, and coughing, sneezing, watery eyes, itching in eyes and nose, headache, and wheezing in people allergic to airborne particles of dust, dander, or plant pollens.

Allergic rhinitis is associated with significant effects on quality of life. Nasal congestion, one of the most common and bothersome symptoms of these conditions, is associated with sleep-disordered breathing and is thought to be a key cause of sleep impairment (see ref. 17).

A rhinitis questionnaire (RQLQ/miniRQLQ) can be used to monitor and assess rhinitis symptoms e.g. during treatment (see ref. 18).

Improvement or reduction of quality of sleep owing to asthma or allergic rhinitis can be monitored by use of questionnaires and objective measures.

In some people, allergen exposure can cause a reaction known as the allergic response. This occurs when allergens are inhaled into the respiratory tract (nose, throat and lungs) and attach to the mucous membranes. These allergens are seen by the immune system as foreign invaders and an immune response is produced as the body prepares to fight them off. During this response, T-cells (a cell type of the immune system) send a signal to B-cells (B-lymphocytes) and stimulate production of IgE antibodies - a key protein involved in the allergic cascade.

IgE antibodies, specific to the allergen, are produced within a few weeks after exposure and released into the bloodstream. These IgE antibodies may attach to receptors on inflammatory cells such as mast cells. Unattached IgE antibodies remain free floating in the bloodstream. When an allergic individual is re-exposed to an allergen, cross-linking to IgE bound on the mast cells may occur (see below).

When cross-linking occurs, mast cells release chemical mediators such as histamine, prostaglandins and leukotrienes (see below). These chemical mediators can cause inflammatory responses in the body. These inflammatory responses have been linked to asthma signs and symptoms such as bronchial constriction, coughing and wheezing.

Devices that reduce exposure to residential airborne contaminants, such as allergens and pollutants, can be useful in residential and institutional settings. Clean air technology is highly effective at removing airborne particles by passing an ambient air-stream through High Efficiency Particulate Air (HEPA) filters. However, the efficiency of HEPA filtration systems depends on airflow dynamics of the environment in which the device is used. In-mixing of contaminated ambient air with filtered air typically diminishes the ultimate efficiency of HEPA filtration, e.g. in providing a purified personal breathing zone.

Several devices have been reported that provide a purified personal breathing zone.

WO2008/058538, US 6,910,961 and US2008/0308106 describe specialized air supply outlets that can be positioned to provide conditioned air for a personal clean-air environment.

US2008/0307970 describes a neck-worn device.

US 6,916,238 describes an enclosed clean air canopy that provides a purified personal breathing zone during sleeping hours.

US 7,037,188 describes a bed ventilation system that provides a purified personal breathing zone during sleeping hours.

US 6,702,662 describes a device that utilizes TLA to provide a personal breathing zone. In this device, filtered air is divided into *two* partial air-streams one of which is cooled, the other heated.

US 2009/0247065 describes devices and methods for improvement of microvascular function using a TLA device delivering a laminar air flow wherein the temperature difference between the filtered air and the ambient air is 0.3 to 3 ºC. However, this usage relates to an entirely different field of treatment, i.e. microvascular function and the device, furthermore, operates using a temperature difference of a much wider interval than that disclosed in the present invention (cf. paragraph 0016 of US 2009/0247065].

WO2004092658 describes a system to improve the air quality in a personal breathing environment. The system includes a blower unit that produces a conditioned air flow, a delivery system that delivers air provided by the blower unit to a person's breathing zone, and a conduit that interconnects the blower unit and the delivery system for directing conditioned air from the blower unit to the delivery system. The delivery system is positioned so that it sends conditioned air around an individual's head and into the individual's personal breathing zone, thereby creating a zone of conditioned air around the individual's head.

Certain of these devices utilize impulse or forced-blown air to induce and maintain a stream of filtered air, enveloping a point of care. These methods and devices are, however, associated with uncomfortable air flow drafts, dehydration and an overall poor control of the filtered air-stream velocity. Further, even where the filtered air-stream is substantially laminar, the sometimes high velocities of forced-blown air inevitably invoke turbulent in-mixing of contaminated ambient air, in the absence of a canopy or enclosure.

Turbulent in-mixing of ambient air can be avoided by utilizing gravity to induce a laminar air flow, rather than impulse or blowing force. The principle of TLA is that a laminar flow is induced by an air-temperature difference between supply air and ambient air at the point of care. A substantially laminar flow of filtered, colder air, having a higher density than ambient air descends slowly, enveloping the breathing zone of a sleeping person. The TLA principle provides an unprecedented ability to control the air flow velocity as measured at the point of care. Parts of or the whole temperature control device may be situated before or after the blower device supplying the laminar air flow.

TLA is based upon boundary control and unidirectional orientation of a laminar air supply structure. Stable flow conditions are maintained by introducing a temperature gradient (negative buoyancy) between the cooled supply air and ambient air in the human breathing zone. Entrainment including turbulent diffusion of ambient air into the laminar supply stream is here limited to a minimum. The filtrated and cooled laminar air, with higher density than ambient air, descends slowly enveloping the breathing zone of a person in bed. This downward directed displacement flow will pass physical obstacles in the air-flow path without a substantial effect on the air flow. A free and isothermal jet flow loses momentum after bouncing off physical obstacles. In contrast, the cooled TLA air retains its lower temperature despite interactions with physical obstacles. TLA thus provides improved removal of contaminants from the breathing zone to the floor level.

It has previously been demonstrated that allergens and other airborne particles accumulate in bedding. In the present invention it has been found that a warm human body lying in a bed causes a convection flow transporting a high concentration of allergens and airborne particles to the person's breathing zone. This phenomenon appears independently of any decrease in particle concentration in the ambient air due to operating room air cleaner. Room air cleaner systems thus cannot typically displace body convection and provide a controlled personal breathing zone.

To be effective in providing a controlled personal breathing zone, a TLA device will ideally provide a substantially laminar descending air flow having sufficient velocity to displace convection currents caused by body heat. A warm human body causes a convection air flow having an ascending velocity of over 0.1 m/s and having an air-temperature increased as much as 2 °C above ambient air at body level. An effective TLA device thus typically provide a descending, substantially laminar flow of filtered air with a velocity higher than 0.1 m/s, and in any case, sufficient to break body convection currents of a sleeping (or similar state) person laying in bed.

Excess velocity of filtered air is, however, undesirable. Excess air flow velocity gives rise to drafts, which are both uncomfortable and, also, dehydrating. Avoiding drafts and dehydration is pivotal for the long term compliance by patients. Bare parts of the human body are extremely sensitive for air movements during low activity or sleep. Furthermore, the greater the velocity of the descending laminar air-stream, the more difficult it is to control and direct it to the point of care without in-mixing of ambient air.

In a TLA device, the velocity of the descending air-stream is determined by the air-temperature difference (i.e. density differences) between the colder, filtered supply air and the ambient air at the level of the point of care. Only minimal impulse is imparted to the air-stream, sufficient to overcome resistance at the outlet nozzle.

Commercially available TLA devices include the Airsonett PROTEXO™, the Airex M100™ and the EIU™.

A number of studies previously made on air cleaning, ventilation and other allergen preventative measures have shown little or no effect on patients with perennial allergic asthma (see ref. 19), suggesting that the allergen reduction has simply not been significant enough to affect the airway inflammation.

Two studies on the effect of air filtration on patients suffering from pet allergic asthma were analyzed by Kilburn *et al.* and it was concluded that HEPA filtration units did not improve the health status of the patients participating in the studies (See ref. 20).

In a study by Sheikh *et al.* it was concluded that previous intervention studies using HEPA filtered air against perennial allergic asthma and allergic rhinitis were not conclusive and further adequately designed trials were required to reach any conclusion on the efficacy of such methods (see ref. 21).

In a more recent publication by the Global Initiative for Asthma it was concluded that conflicting evidence exists as to whether measures to create low-level allergen environments in patient's homes and reduce exposure to indoor allergens are effective at reducing asthma symptoms. Also, that the majority of single interventions have previously failed to achieve a sufficient reduction in allergen load to lead to clinical improvement and that no single intervention will achieve sufficient benefits to be cost effective. However, in one study comprehensive indoor environmental intervention, including mandatory instructional home visits by researchers, use of allergen-impermable covers, air filters and vacuum cleaners having HEPA filters and professional pest control has been shown to reduce allergen load and reduce asthma-associated morbidity (see ref. 22, page 55).

The use of a TLA device in the treatment of asthma and allergy is disclosed in a marketing brochure published by the company Airsonett AB. The brochure reproduces data from two case reports on individual asthma patients and a pilot study comprising 7 allergic adults.

The use of TLA in the treatment of asthma is also described by Pedroletti *et al.* wherein a double-blind, placebo-controlled, cross-over study showed that the use of a TLA device add-on treatment in 22 patients with perennial allergic asthma, taking regular ICS ≥200µg/day of budesonide or 100µg/day of fluticasone, produced a significant increase of Quality of Life (based on questionnaire score) and decrease of airway inflammation (based on fractional nitric oxide in exhaled air) after ten weeks of treatment, compared to placebo. It is, however, concluded that the results require verification in a larger clinical trial before any general treatment recommendations can be made (see ref. 23).

### Brief description of the figures

Figure 1 illustrates convection currents generated by a warm body in a sleeping position.
Figure 2 illustrates a controlled personal breathing zone generated by TLA.
Figure 3 illustrates an embodiment of a device according to the invention.
Figure 4 illustrates embodiments of filtered air-stream temperature adjustment units.
Figure 5 illustrates alternative systems for dissipation of excess heat from the air-stream temperature adjustment unit.
Figure 6 illustrates functioning of one embodiment of a nozzle.
Figure 7 illustrates some alternative arrangements of preferred embodiments used in providing a controlled personal breathing zone useful for the treatment of asthma.
Figure 8 illustrates a time line for the clinical study referred to in example 1.

### Summary of the invention

The current invention relates to a temperature controlled laminar airflow (TLA) device for supplying a substantially laminar airflow directed to the breathing zone of a patient suffering from asthma during situations of or corresponding to sleep whereby allergen avoidance is achieved for a significant period of the day whereby symptoms of asthma are reduced or removed. The TLA treatment is designed to significantly reduce the allergen load in the patient's breathing zone by vertically displacing the contaminations, originating from the bed and the room environment, with a laminar, allergen free, airflow during sleep. The airflow is filtered, slightly cooled and showered over the patient. Due to the higher density, the cooled air descends slowly down, and displaces the contaminants from the breathing zone. In a controlled clinical trial it has surprisingly been found that air 0.5 to 1 °C cooler than the ambient air effectively displaces warm body convection currents without causing an unpleasant draft whereby symptoms of allergic asthma and allergic rhinitis are reduced. Furthermore, quality of sleep can be improved.

### Detailed description of the invention

Devices for providing a controlled breathing zone are known in the art, but none of these have been described to effectively reduce or remove symptoms of asthma and rhinitis without imposing an unpleasant draft onto the patient using the device. Also a patient's quality of sleep can be improved by use of a device according to the present invention.

The term asthma includes perennial asthma, the term rhinitis includes perennial rhinitis and the term allergy includes perennial allergy.

The present invention provides an easy-to-use non-invasive device for reducing or removing symptoms of asthma and rhinitis in asthma patients wherein the word patient in this context is taken to mean any person diagnosed with asthma, allergic asthma and/or allergic rhinitis including periodically non-symptomatic persons. The present invention also provides uses of said device for improving quality of sleep in said patient.

In one embodiment, the invention provides a device for reducing or removing the symptoms of asthma and/or rhinitis in a patient in need thereof by reducing exposure to allergens and other airborne fine particles as measured at the point of care by delivering treated air into a zone around the patient generating a treated air zone wherein said treated air descends in a laminar fashion thereby substantially preventing in-mixing of ambient air characterized in that the air-temperature of the air delivered into the treated air zone is 0.5 to 1 °C cooler than the ambient air (such as 0.5 to 0.9 °C or 0.6 to 0.8 °C cooler) surrounding the treated air zone whereby warm body convection currents are displaced without the patient being exposed to unpleasant draft.

In another embodiment, the invention provides a device for improving quality of sleep in a patient in need thereof by reducing exposure to allergens and other airborne fine particles as measured at the point of care by delivering treated air into a zone around the patient generating a treated air zone wherein said treated air descends in a laminar fashion thereby substantially preventing in-mixing of ambient air characterized in that the air-temperature of the air delivered into the treated air zone is 0.5 to 1 ºC cooler than the ambient air (such as 0.5 to 0.9 °C or 0.6 to 0.8 °C) surrounding the treated air zone whereby warm body convection currents are displaced without the patient being exposed to unpleasant draft.

In one embodiment the patient receives a daily maintenance dose of corticosteroids.

In other embodiments, the invention provides devices for displacing body convection and providing a controlled personal breathing zone. Specifically an air-treatment device having at least one filter for reducing or removing the symptoms of asthma in a patient in need thereof by reducing exposure to allergens and other airborne fine particles by delivering treated air into a zone around the patient generating a treated air zone wherein said treated air descends in a laminar fashion thereby substantially preventing in-mixing of ambient air characterized in that the air-temperature of the air delivered into the treated air zone is 0.5 to 1 °C cooler than the ambient air surrounding the treated air zone whereby warm body convection currents are displaced without the patient being exposed to unpleasant draft.

In a preferred embodiment the device is used to reduce or remove the symptoms of asthma such as perennial allergic asthma in a patient in need thereof.

In another preferred embodiment the device is used to remove the symptoms of rhinitis such as allergic rhinitis in a patient in need thereof.

In yet another preferred embodiment the device is used to improve quality of sleep in a patient in need thereof.

In one embodiment a patient experiences a controlled breathing zone during sleeping hours that is associated with minimal operating noise generated by the device. In one such embodiment a patient is treated for an extended period of time such as - but not limited to - 2 weeks or 3, 6 or 12 months or for the remainder of the patient's life.

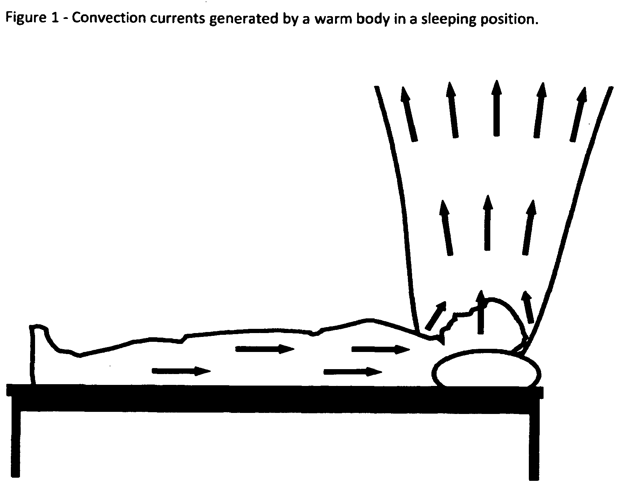

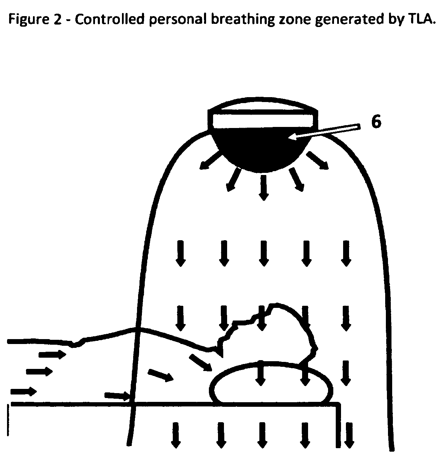

As shown in Figure 1, the warm body of a patient in a sleeping position generates convection air currents. To be effective in providing a controlled personal breathing zone, TLA devices of the invention preferably provide a descending stream of filtered air that has sufficient velocity to overcome these convection body currents, as shown in Figure 2 without imposing an unpleasant draft on the patient.

In preferred embodiments, a device according to the invention utilizes TLA to generate a descending and substantially laminar flow of filtered air. This provides a controlled personal breathing zone that is substantially free of in-mixed, contaminated ambient air, while displace body convection. The zone of treated air provided by such devices may provide more than 95% reduction in airborne fine particle counts, and typically provide at least more than 75% reduction at the point of care. In one embodiment more than 95% of particles larger than 0.5 µm are removed.

In one embodiment the device according to the invention reduces breathing zone cat allergen concentration by a factor of 30 and total breathing zone particulate exposure by a factor of 3000 for particles >0.5 µm and 3700 for particles >10 µm. Thus, effectively reducing aeroallergen such as pet dander (predominately < 5 µm) and house dust mites (>10 µm) exposure at the point of care.

In a preferred embodiment the temperature difference between the air that is delivered and the ambient air is maintained substantially constant.

In yet another preferred embodiments the treated air zone is limited to the head area of the patient.

A suitable device comprises at least one of each of the following: (1) an air inlet, (2) a filter, (3) a blower, (4) an air-temperature adjustment system, (5) an air-temperature control system, (6) an air supply nozzle, and (7) a housing.

The one or more air inlets (1) are preferably placed near the floor level of the premises in which the device is utilized, where the layer of coolest air is situated. Alternatively air inlets may be placed higher up in the room, although this typically results in higher energy consumption in that warmer layers of air must be cooled. Preferably, the air inlets are configured in such manner as to keep emission of sound waves during operation to the lowest practicable levels. The more openings included in the device housing, the greater will be the noise levels perceived by the patient. In some embodiments, the air inlets may be associated with a pre-filter that also serves as a sound damper. In other embodiments, a HEPA filter that provides ultimate filtration of the supply air may be situated directly at the air inlets.

The filter (2) is preferably a high efficiency particulate air filter, preferably HEPA class H11, or higher if needed at point of care. In other embodiments, any suitable filter media or device adapted to filter particles or gases unwanted at the point of care may be used. Including for example any combinations of fiberglass and/or polymer fiber filters, or electro static filters, or hybrid filters (i.e. charging incoming particles and/or the filter media), or radiation methods (i.e. UV-light), or chemical and/or fluid methods, or activated carbon filters or other filter types.

While filter effectiveness is preferably high and stable over time, the resistance to air flow, or "pressure drop" generated by the filter is preferably kept low. Increased pressure drop generated by the filter, the device housing, the air delivery nozzle and other components and air channels of the device calls for increased blower speed which in turn generates unwanted noise. In preferred embodiments, pressure drop of a suitable filter is generally lower than 50 Pa. When using the preferred embodiment of HEPA filter using fiberglass or polymer fiber filter media, pressure drop is generally minimized by maximizing the active filter media area.

In preferred embodiments, HEPA filters are comprised of randomly arranged fibers, preferably fiberglass, having diameters between about 0.5 and 2.0 micron, and typically arranged as a continuous sheet of filtration material wrapped around separator materials so as to form a multi-layered filter. Mechanisms of filtration may include at least interception, where particles following a line of flow in the air-stream come within one radius of a fiber and adhere to it; impaction, where large particles are forced by air-stream contours to embed within fibers; diffusion, where gas molecules are impeded in their path through the filter and thereby increase the probability of particle capture by fibers. In some embodiments, the filter itself may comprise the air supply nozzle through which supply air is delivered.

Alternatively or complementary to a HEPA filter, any suitable air treatment system can be used, including at least a humidifier or a dehumidifier, ionizer, UV-light, or other system that provides air treatment beneficial at the point of care.

Preferred embodiments of a device according to the invention comprise an electronic filter identification system. When a filter becomes clogged with particles, its effective area is decreased and its pressure drop accordingly increased. This results in lower airflow, which reduces overall effectiveness of the device. Accordingly it is preferable that patients change the filter within the recommended service interval. To facilitate proper use, preferred embodiments provide a filter management system that indicates when a filter should be changed. Each filter can be equipped with a unique ID that permits the TLA device to distinguish previously used filters from unused ones. Filter identification systems can be provided RFID, bar codes, direct interconnections, attachments such as iBUTTON™ circuits on a circuit board on the filter. It might also be possible to read or read and store other data than the serial number on the filter by this system. Information about the most appropriate airflow according to the filter type can for instance be supplied with the filter and be read automatically by the system.

The blower (3) generates air flow needed to feed a sufficiently large stream of air and to create pressure sufficient to overcome the pressure drop generated by the device. The blower may be of any suitable design, preferably comprising a fan impeller/blower rotor driven by an electric motor. Preferred embodiments are adapted so as to generate minimal noise during operations.

Blower noise is generally minimized by maximizing the size of the rotating rotor and minimizing the rotation per minute.

In preferred embodiments the fan generates a flow of filtered air through the device is less than 500 m³/h, such as less than 400 m³/h, preferably less than 300 m³/h, such as less than 250 m³/h, more preferably less than 225 m³/h, such as less than 200 m³/h, and even more preferably less than 175 m³/h, such as less than 150 m³/h.

The temperature adjustment system (4) cools and/or warms the supply air. In preferred embodiments, both heating and cooling are provided by a thermoelectric Peltier module. As is known in the art, a Peltier module can provided both heating and cooling depending on the polarity of the applied voltage or the direction of its operating current. In some embodiments, heating can be provided by an electric radiator, an electric convector or other type of heating methods, while cooling is provided by compressor (i.e. by using the Carnot process), or by fresh water cooling or other cooling means.

The temperature adjustment system preferably generates as little pressure drop as possible, preferably it has sufficiently large emission surfaces so as to avoid unwanted condense water when cooling in warm and humid conditions, and is preferably able to maintain a cooling power that is stable over time and with minimal short term variations of supply air-temperature.

In preferred embodiments, heating/cooling is evenly distributed by means of heat pipes. Fins mounted on the heat pipes, with short distance to heat/cool source, can cover a wide cross section area of the air flow. Because the distance to the heat/cool source is short, efficient heat exchange can be achieved using relatively thin fins. In contrast, relatively thicker fins with lower thermal resistance are required using extruded heat sinks because of the longer distance to the heat source. Accordingly, the heat pipe system can effectively provide heat/cool transfer to a cross section area of air flow with comparably thinner fins resulting in lower air resistance and minimized pressure drop. Further, the short distance to the heat/cool source using heat pipes leads to an evenly distributed surface temperature which makes more efficient heat transfer per unit fin area. This leads to smaller temperature differences and thereby less risk of condense water accumulating on cooler areas of the fins.

It will be readily understood by one skilled in the art that a variety of different schemes for temperature adjustment may be employed. In systems that utilize a thermoelectric cooler (TEC), excess heat can be dissipated in variety of ways, including passive or active convection or active liquid cooling.

Preferred embodiments can stably maintain an air-temperature difference of supply air relative to ambient air at the level of the point of care with a minimal fluctuation. Fluctuation of the air-temperature difference is preferably kept within the range of the margin of measurement error, preferably ±0.1 °C. This stable air-temperature difference is preferably maintained at some point within the range of about 0.5 to 1 °C. In this manner, descending air-stream velocity can be "delicately balanced" between excessive velocity, which creates unwanted drafts, and sufficient velocity, which is just enough to break body convection currents.

The temperature control system (5) maintains a stable air-temperature difference between the descending supply air-stream enveloping the point of care (i.e. the breathing zone of a sleeping person) and the ambient air as measured at the level of the point of care. In one preferred embodiment, the temperature control system comprises two sensors and a control unit. One temperature sensor is placed in the supply air channel just after the temperature adjustment device (4). A second sensor is placed in such manner as to measure ambient air at the level of the personal breathing zone but outside the effective stream of supply air. The control unit is preferably programmed to collect data from the two sensors and to regulate voltage applied to the Peltier element so as to maintain a temperature difference within the optimal range. Sensors are preferably protected from any kind of radiation from surfaces so as to provide an accurate air-temperature measurement. Preferably, sensors have high sensitivity and minimal error margin, ±0.05 °C.

The air supply nozzle (6) delivers a substantially laminar stream of supply air with minimal in-mixing of ambient air. In order that velocity of the supply air-stream may be determined by difference in air-temperature from ambient air at the level of the point of care, supply air preferably exits the nozzle with velocity (i.e., dynamic pressure) that is just sufficient to overcome nozzle resistance. This initial dynamic pressure of supply air is rapidly diminished by static pressure of ambient air until a point is reached at which gravity alone (i.e. air-temperature difference) determines the rate of further descent. The nozzle preferably has minimal resistance whereby supply air may exit the nozzle with minimal dynamic pressure and, accordingly, whereby the point at which air-temperature difference alone determines the rate of further descent is reached well before the supply air-stream reaches the point of care. In some embodiments, the nozzle (6) can be replaced by or made in combination with one or more filters (2) as an integral part of the air supply nozzle or as the sole part delivering supply air.

A wide variety of nozzle shapes and sizes can be used. However, the rate at which initial velocity of supply air is diminished by static pressure of ambient air is affected by nozzle shape. Pitch length refers to the distance from the surface of the nozzle at which the cumulative effect of static pressure of ambient air counterbalances the dynamic pressure of supply air that has been set into flow with impulse just sufficient to overcome resistance in the nozzle. Preferably, a suitable nozzle has minimal pitch length. This permits gravity (i.e., air-temperature difference) to control the downward air flow velocity at a point well above the point of care. Short nozzle pitch length also ensures that supply air flow will introduce minimal disturbance of ambient air which in turn minimizes turbulences that arise when supply air meets still, standing ambient air. In preferred embodiments, nozzle pitch length ends well before the point of care.

Preferably the pitch length, as defined by an air velocity of less than 0.2 m/s, should reach less than 20 cm from the air delivery device. In any case, the pitch length is preferably no longer than the distance between the air supply nozzle and the point of care. The prime factors determining the actual pitch length are shape of the nozzle and the composition the materials shaping the nozzle. A preferred nozzle is described in WO2005/017419, which is hereby incorporated by reference in its entirety. An air delivery nozzle with a substantially spherical shape as described is likely to cater for a larger effective operative area as compared to a flat air delivery nozzle, given identical air flow. However, both flat or spherical shaped nozzles can be used.

The substantially spherical shape has the advantage of being compact. Further the shape forces the air flow to be distributed over an increasing surface area. This reduces pitch length, in that the decrease in air velocity is dependent on friction between the supply air and ambient air. The spherical surface distributes supply air flow to a surface are that increases with approximately the square of the distance from the nozzle centre. The increasing surface area forces the velocity to decrease with approximately 1 /(the square of the distance from the nozzle centre) giving the spherical nozzle a natural character with a short pitch length. In contrast, a flat delivery nozzle generates an air flow with a constant distribution area and a correspondingly longer pitch length.

Any alternative nozzle with similar characteristics of minimal pitch length and low disturbance of ambient air may be used.

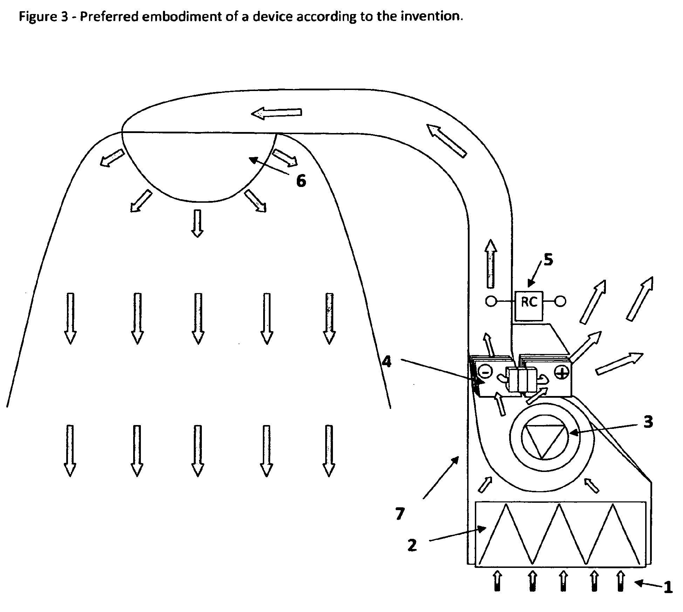

Figure 3 illustrates a preferred embodiment of a device according to the invention. Ambient air (symbolized by shaded arrows, indicating flowing air) is taken in through the air inlet (1), which is situated at floor level at the bottom of the housing (7). Intake air is filtered by the filter (2), driven by action of the blower (3). An air-temperature adjustment device (4) is situated so as to provide both cooling and heating of the filtered supply air-stream. The device comprises a Peltier element with reversible voltage polarity connected via heat pipes to two sets of fins. One set of fins serves primarily to distribute cooling effect in the supply air-stream while the other set of fins serves primarily to provide dissipation of excess heat generated by the Peltier module. Parts of or the whole air-temperature adjustment device (4) may be situated before the filter (2) and/or the blower (3). Parts of or the whole air-temperature adjustment device (4) may also be situated in other parts of the device such as the nozzle (6). The temperature control device (5) comprises a control unit (square) and two sensors (circles). One sensor is placed in the supply air-stream while the other is placed in such manner so as to measure ambient air-temperature at the level of the personal breathing zone but outside the supply air-stream. The control unit, informed by air-temperature measurements from the sensors, regulates the temperature adjustment unit so as to maintain a stable air-temperature difference between the supply air and ambient air at the level of the point of care. Supply air is driven by action of the blower (3) out of the nozzle (6) with minimal impulse.

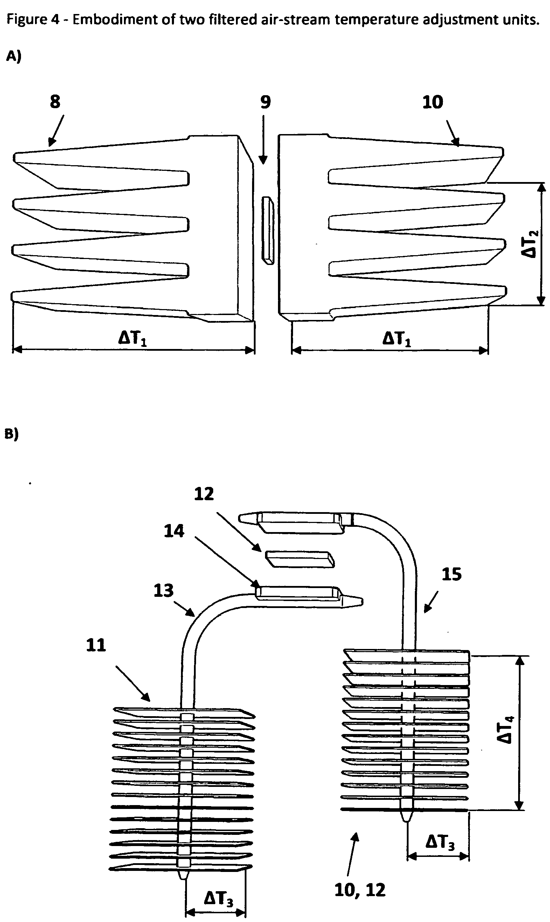

Figure 4 shows, in greater detail, an air-temperature adjustment unit (4) of a preferred embodiment. Figure 4A shows a TEC system with extruded heat sinks. In this system the TEC (9) distributes generated cooling effect on one side by interfacing an extruded heat sink (8). On the other side of the TEC heat is dissipated to a similar extruded heat sink (10). Figure 4B shows a heat pipe system. Here the TEC (12) interfaces a connection block (14) with at least the same area as the TEC. From here the cooling effect is transported to the fins by a heat pipe (13). At the warm side (15) the heat is transferred in the same way. The Peltier element is normally fitted with thermal grease or a thermal pad which increases the thermal conductivity of the thermal interface by compensating for the irregular surfaces of the components.

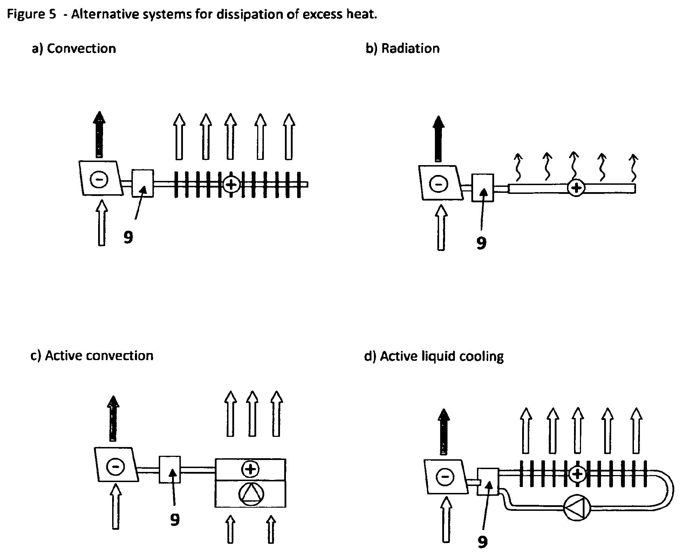

Figure 5 shows alternative systems for dissipating excess heat generated by the air-temperature adjustment unit. In a preferred embodiment using a TEC system, excess heat can be dissipated by convection, as shown in Figure 5a, by radiation, as shown in Figure 5b, by active convection, as shown in Figure 5c, or by active liquid cooling, as shown in Figure 5d. These alternative systems may act alone or in combination (i.e. by combining convection with radiation).

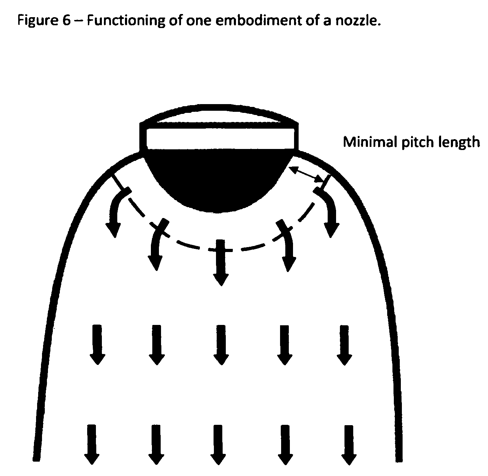

Figure 6 illustrates functioning of the nozzle (6) of the preferred embodiment shown in Figure 3. Shown is a schematic illustration of the functioning of the nozzle described in WO2005/017419.

Supply air is initially forced out of the nozzle with a slight velocity, about 0.2 m/s, just sufficient to overcome resistance in the nozzle. The spherical surface distributes supply air flow to a surface area that increases with approximately the square of the distance from the nozzle centre. Friction with ambient air dissipates the air flow velocity up to the pitch length, after which further descent of the supply air-stream is determined by air-temperature difference (gravity).

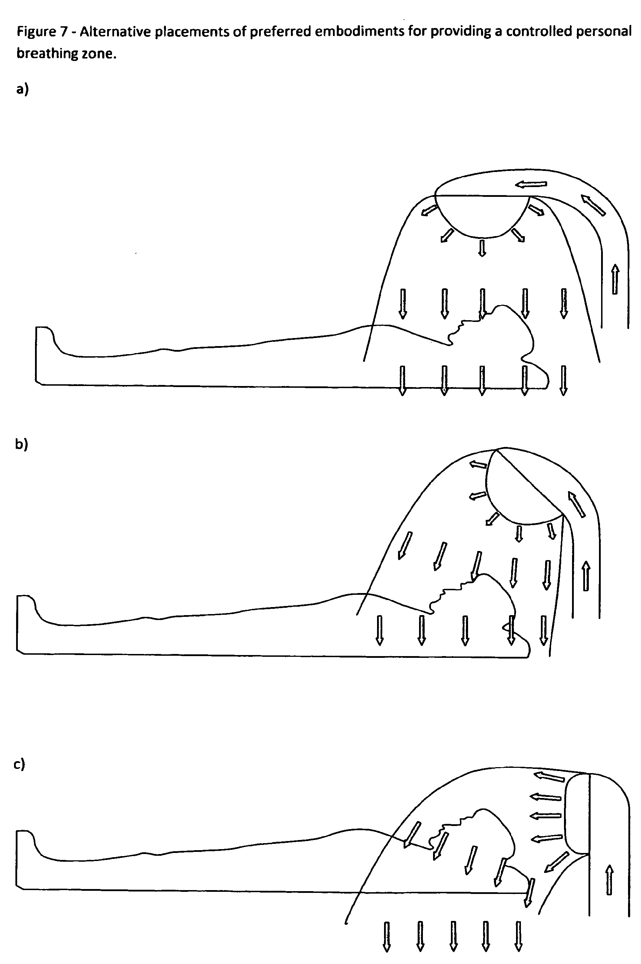

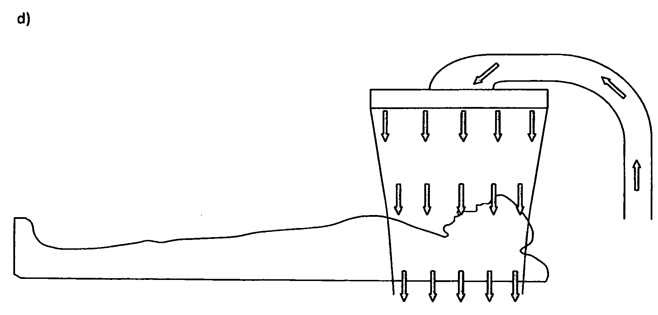

Figure 7 illustrates some alternative arrangements of preferred embodiments used in providing a controlled personal breathing zone. The air delivery nozzle, which can be spherical or flat or other shape, can be placed straight above the point of care, as shown in figures 6a and 6d. It can be slightly tilted and placed slightly off center of the point of care, as shown in figure 6b. It can be placed aside the point of care directing an impulse horizontally towards the point of care, as shown in figure 6c. In all settings gravity (temperature difference) defines a substantially downward directed air-stream (after initial forced impulse has been counteracted by friction with ambient air). The downward directed supply air-stream has sufficient velocity to displace conflicting body convection as illustrated in Figure 1. The preferred distance between the nozzle and the point of care is preferably within the range of about 20 cm to 80 cm.

In an unpublished multiple independent, double blind, randomized 52 week parallel clinical trial comparing active and placebo treatment with a TLA device, using one embodiment of the TLA device described in US 6,702,662, we have found that a relatively narrow range of conditions exists in which it is possible to reduce or remove the symptoms of asthma while avoiding drafts (caused by excessive velocity of the descending air-stream) while also avoiding the inability to displace warm body convection currents (caused by insufficient velocity of the treated air-stream) and at the same time minimizing in-mixing of ambient contaminated air.

The use of a TLA device in the treatment of asthma and allergy is disclosed in a marketing brochure published by the company Airsonett AB. In terms of asthma the brochure reproduced data from two case reports on asthma patients. Also, treatment of asthma using a TLA device is described in a publication by Pedroletti *et al.* (see ref 21). However, no information is provided as to optimum temperature difference between the treated air supplied and the ambient air nor is any information provided on how to displace warm body convection currents or avoid draft caused by the air flow. Additionally, none of the studies are powered to substantiate clinical recommendations. Indeed the brochure published by Airsonett AB concerns one study having as little as 7 participants and two case reports based on just 1 subject, respectively.

The Pedroletti *et al.* study included more test subjects, however, it is stressed in the publication that a larger trial is required to make clinical recommendations (see ref. 21).

In the study upon which the present invention is based it was surprisingly found that an optimum air-temperature difference between the filtered, descending laminar air and the ambient air at the level of the personal breathing zone falls within a range of about 0.5 to 1.0° C. This study confirmed that treatment with a TLA device on top of ICS medication effectively leads to a decreased airway inflammation.

In preferred embodiments, a single filtered air-stream is subject to temperature adjustment and air-temperature of the filtered air can be carefully adjusted via a temperature control system to maintain, within the optimum range, an air-temperature difference between supply air and ambient air at the level of a personal breathing zone. Reversible polarity of the TEC used to provide air-temperature adjustment permits the supply air-stream to be alternately cooled or heated, thereby providing necessary fine tuned control of descending air-stream velocity.

The impact of non-compliance with prescribed treatments on healthcare costs is huge. It has been estimated for the US alone at between $ 77 billion and $300 billion per annum (depending on whether it accounts for direct costs only or includes indirect costs such as lost productivity as well).

Patient compliance is a major issue in the long term treatment of chronic diseases. Patient non-compliance with a prescribed drug regimen could result in ineffective therapy management with possible dangerous health consequences. In the case of certain prescription drugs used for chronic diseases such as asthma, non-compliance may lead to frequent hospital emergency department visits (about 1.8 million p.a. in the US, 2001) or even patient deaths (4,200 patients died from asthma attack in the US in 2002). Further consequences for the patient are also increased morbidity, treatment failures, exacerbation of disease and more frequent physician visits. Apart from this society will incur costs from absenteeism and lost productivity at work. Hence there is an unmet need for treatments where compliance can be easily monitored thus saving healthcare costs while increasing patients' clinical outcome and quality of life

After regulatory approval by FDA and EMEA or issue of CE mark by competent bodies the access to the subsidized market is today still regulated as a number of gatekeepers today ask for a comprehensive package of evidence to support the access to the market as a reimbursed product.

The gatekeepers range from national ones such as the French National Authority for Health (CNEDiMTS), the National Institute of Clinical Excellence (NICE) in England and Medicare in the US to regional ones such as Medicaid in the US, Statutory Health Insurances (krankenkassen) in Germany, County Councils (landstingen) in Sweden and Primary Care Trusts (PCT) in England.

To adhere to the requirements of said gatekeepers - which is letting only products offering value for money into reimbursement schemes - the TLA device has been subject to a randomized, double blind placebo controlled study. The study has generated new and innovative results that will be submitted as part of a health economic model to the relevant authorities allowing for reimbursement. The use of this strategy for conducting its business at both national and international level is considered new and useful.

### Example 1: Clinical study relating to treatment of asthma

To compare the efficacy of the Airsonett Airshower (AA) TLA device with a placebo device to reduce the degree of asthma symptoms in patients with perennial allergic asthma, sensitised to animal dander and/or house dust mites a clinical study was carried out.

The primary endpoint of the clinical study was a mini-AQLQ/PAQLQ score reflecting the developments in the symptoms of asthma. The mini-AQLQ/PAQLQ instrument is generally accepted as being sufficiently simple and robust to be suitable for research and quality of care monitoring in primary care at the group level.

As a secondary endpoint the efficacy of the AA compared with a placebo device to decrease FENO and increase PEF and FEV₁, rhinitis symptoms (nasal block, rhinorrhea and sneezing), and quality of sleep was compared. Furthermore, the efficacy to reduce the RAST/ImmunoCAP value, i.e. allergen specific IgE levels and the eosinophil count from study start to study termination was investigated.

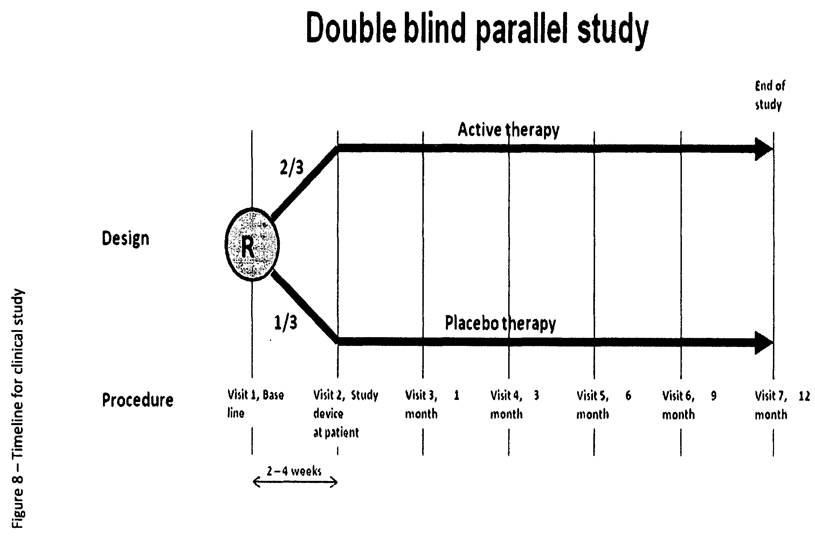

The study was carried out as a multiple independent, double blind, randomized 52 week parallel trial comparing active and placebo treatment with the AA TLA device. For ethical reasons the randomization of patients was 2 to 1 for active and placebo treatment, respectively. Patients were randomized and included at visit 1 and a device was installed 2-4 weeks after inclusion. During this run-in period the patient could become familiar with the use of the patient asthma diary and how to adhere to the requirements of the study participation. For the first 3 months an unchanged maintenance medication was maintained and month 4 - 12 medication was based upon GINA (Global Initiative for Asthma) 2006 guidelines. At inclusion baseline measurements were evaluated and then active/placebo treatment with AA was implemented over 52 weeks. See the timeline in figure 8 for a time line overview of the study.

The patient population consisted of male and female patients, 7 to 70 years of age, with established asthma and documented allergy to one or more of allergens:

Animal dander and/or house dust mites, and maintained on 200-1200 µg/day budesonide or equipotent inhaled corticosteroid (ICS). At inclusion the patients should have one or more features of partly controlled asthma according to GINA 2006. Patients should also have a maximum score of ≤ 5.5 measured with mini-AQLQ/PAQLQ at inclusion.

AA active and AA placebo devices were used as test articles. In the placebo devices the filter was bypassed and the cooling turned off which prevented the air from reaching the breathing zone. The devices were installed by an independent person appointed by the Airsonett AB and sealed. No clinic personnel or trial monitor had access to the randomization list.

The procedure followed in the clinical trial was thus as follows: After inclusion and baseline measurements active/placebo treatment with AA was implemented over 52 weeks having the study parameters: Total mini-AQLQ/PAQLQ, FENO, FEV₁, PEF, FEF₅₀ (forced expiratory flow rate at 50% of the vital capacity), ACT, allergen specific IgE, number of exacerbations, hospitalization and rhinitis symptoms (RQ) by age, sex and severity of asthma at baseline.

Data analysis consisted of statistical calculations useful for comparing significant differences between the active and placebo treatment with respect to:
Difference in mini-AQLQ and PAQLQ scores, FENO value, FEV₁, PEF and FEF₅₀. Difference in number of Exacerbations, specific IgE, use of ICS, SABA and LABA, hospitalization, rhinitis symptoms, lost school/workdays and Quality of sleep.

Difference in AQLQ (mini-AQLQ and PAQLQ) score between visit 7 and visit 1 (see figure 8) was evaluated using analysis of covariance (ANCOVA), adjusting for treatment, baseline score, age, medical history and sites and using the LOCF technique. Improvement in quality of sleep between visit 7 and visit 1 was evaluated in the aforementioned method based on a specific question in the mini-AQLQ.

Difference in FENO between visit 7 and visit 1 (see figure 8) was evaluated using analysis of covariance (ANCOVA), adjusting for treatment, value at baseline, age, medical history and sites and using the LOCF technique.

The trial was performed in accordance with the recommendations guiding physicians in biomedical research involving human Patients adopted by the 18th World Medical Assembly, Helsinki, Finland, 1964 and later revisions, ICH guidelines and good clinical practice.

In evaluating the efficacy of the asthma treatment in the individual patient a minimum score improvement of at least 0.5 in the mini-AQLQ or PAQLQ was required in order to consider the patient a success. A subpopulation diagnosed with allergic rhinitis was part of the patient group allowing for evaluation of the efficacy of TLA treatment in this population. Patients reporting an improvement of less than 0.5 (and reductions in score) were considered failures. Changes in the mini-AQLQ and PAQLQ scores were monitored during the study (see figure 8). For the intention to treat (ITT) patient population the improvement in scores obtained after 12 months compared to the baseline are shown in table 1. It can be concluded that the percentage of patients in TLA treatment group having a score improvement of at least 0.5 is significantly higher than the percentage of patients in the placebo group having a score improvement of at least 0.5, i.e. that symptoms of asthma are reduced.

**Table 1 - Improvement in overall mini-AQLQ/PAQLQ responder rate**

| | |
|---|---|
| Difference in responder rates as measured in %-units (in patients having an mini-AQLQ/PAQLQ score increase ≥0.5) between TLA and placebo treatment | 14.8% |
| p-value* | p = 0.02 |

| | |
|---|---|
| * Statistics were carried out using Last Observation Carried Forward to impute missing values | |

A positive correlation was observed in patients being sensitized to several allergens (i.e. having more than 1 allergy) and the effect of TLA treatment on mini-AQLQ/PAQLQ responder rate after 12 months of treatment.
Further to the results shown in table 1, an increasing number of patients in sub-populations having more than 1 or 2 allergies had a score improvement of at least 0.5. Thus, the TLA treatment described in the present inventions appears to be increasingly effective in patients suffering from multiple types of allergy (cf. Table 2).

**Table 2 - Improvement in overall mini-AQLQ/PAQLQ responder rate**

| | |
|---|---|
| Difference in responder rates as measured in %-units (in patients having a mini-AQLQ/PAQLQ score increase ≥0.5) between TLA and placebo treatment in patients with >1 allergies. | 18% |
| | p = 0.009* |
| Difference in responder rates as measured in %-units (in patients having a mini-AQLQ/PAQLQ score increase ≥0.5) between TLA and placebo treatment in patients with >2 allergies. | 26% |
| | p = 0.014* |

| | |
|---|---|
| * Statistics were carried out using Last Observation Carried Forward to impute missing values | |

An analysis of the primary efficacy dataset using alternative methodology (Mixed Models, without imputation of missing data) demonstrated a significant interaction between baseline asthma treatment intensity and the effect of TLA treatment on mini-AQLQ/PAQLQ score, with patients at GINA treatment step 4 (i.e. patients receiving more controller medication) having increased treatment effect compared with patients at GINA steps 2 or 3 (cf. ref. 22, p. 59).

The level of treatment intensity could also be correlated with the efficacy of TLA treatment as a higher percentage of patients with poorly controlled asthma receiving high intensity medication (GINA step 4 patient sub-group) achieved a mini-AQLQ/PAQLQ score increase of ≥0.5 compared to the corresponding patient population receiving placebo treatment (cf. table 3).

**Table 3 - Improvement in overall mini-AQLQ/PAQLQ responder rate**

| | |
|---|---|
| Difference in responder rates as measured in %-units (in patients having a mini-AQLQ/PAQLQ score increase ≥0.5) between TLA and placebo treatment in GINA step 4 patients with poorly controlled asthma. | 25.4 % |
| | p = 0.009* |

| | |
|---|---|
| * Statistics were carried out using Last Observation Carried Forward to impute missing values | |

Changes in FENO levels were monitored during the study (see figure 8). For the ITT patient population the changes in FENO levels obtained after 12 months compared to the baseline are shown in table 4. It can be concluded from the data that TLA treatment results in a significant improvement in FENO levels when compared to the placebo group.

**Table 4 - Improvement in FENO levels**

| **Treatment type** | **Mean change (SEM)** | **p-value*** |
|---|---|---|
| TLA | - 6.19 (2.21) | p = 0.02 |
| Placebo | + 2.29 (3.08) | |

| | | |
|---|---|---|
| * Statistics were carried out using Observed Case | | |

Improvement in quality of sleep was monitored during the study (see figure 8). For the ITT patient population (12 years of age or older) the improvement in quality of sleep obtained after 12 months compared to the baseline are shown in table 5. It can be concluded from the data that TLA treatment results in a significant improvement in quality of sleep when compared to the placebo group.

**Table 5 - Improvement in quality of sleep mini-AQLQ responder rate**

| | |
|---|---|
| Difference in responder rates as measured in %-units (in patients having a mini-AQLQ score increase ≥0.5) between TLA and placebo treatment | 19.5% |
| p-value* | 0.004 |

| | |
|---|---|
| * Statistics were carried out using Last Observation Carried Forward to impute missing values | |

Symptoms in a patient subpopulation suffering from allergic rhinitis were monitored during the study (see figure 8). For the ITT patient population the improvement in AQLQ responder rate obtained after 12 months compared to the baseline are shown in table 6.

**Table 6 - Improvement in allergic rhinitis patient mini-AQLQ/PAQLQ responder rate**

| | |
|---|---|
| Difference in responder rates as measured in %-units (in patients having a mini-AQLQ/PAQLQ score increase ≥0.5) between TLA and placebo treatment | 15.9% |
| p-value* | 0.02 |

| | |
|---|---|
| * Statistics were carried out using Last Observation Carried Forward to impute missing values | |

There is a greater difference between treatment groups in the allergic rhinitis population compared to the whole population (15.9 vs.14.8%-units, cf. table 1), demonstrating that there is a beneficial effect in patients with allergic rhinitis.

Furthermore, this effect has been observed in specific patient groups including patients having treatment intensities 3 and 4 according to GINA guide lines (see ref. 22).

Thus, it is surprisingly found that symptoms of allergic asthma and allergic rhinitis can be reduced and quality of sleep improved using a TLA device delivering treated air at the point of care at a temperature 0.5 to 1 °C lower than that of the ambient air without exposing the patient to an unpleasant draft while resulting in minimal in-mixing of contaminated ambient air.

Asthma can be classified as atopic or non-atopic according to the presence or absence of IgE antibodies to common allergens such as those present in house dust mites and pet dander. Surprisingly, TLA treatment for 12 months prevented rises in aeroallergen-specific IgE. Table 7 below shows the ImmunoCAP results from the clinical study for cat and *Dermatophagoides farinae* allergens. It follows that TLA treatment using a TLA device delivering treated air at a temperature 0.5 to 1 °C lower than that of the ambient air at the point of care without exposing the patient to an unpleasant draft while resulting in minimal in-mixing of contaminated ambient air can significantly decrease IgE levels (or suppress the increase in IgE levels).

**Table 7 - Effect of TLA treatment on allergen specific IgE levels**

| | **Active** | **Placebo** | **p-value**** |
|---|---|---|---|
| Δ *D. farinae** specific IgE (age<12 years) | -5 (-12, 1) | 34 (-10, 77) | 0.01 |
| Δ Cat specific IgE | 8 (0, 17) | 35 (18, 53) | 0.01 |

| | | | |
|---|---|---|---|
| * *Dermatophagoides farinae* is also known as the American house dust mite. ** Statistics were carried out using Observed Case | | | |

Moreover, there was a significant difference between groups in the symptom domain of mini-AQLQ/PAQLQ, with a mean 0.31 point (95% CI 0.01, 0.61) increase after active versus placebo treatment; 0.70 (95% CI 0.13, 1.26) in the subgroup with high treatment intensity and poor symptom control at baseline.

Also, a subgroup of patients suffering from perennial allergic rhinitis and categorized as GINA step 4 patients with uncontrolled asthma experienced either an improvement (55% for TLA treatment vs. 27% for placebo) or no change (45% for TLA treatment vs. 50% for placebo) in activity limitations due to rhinitis symptoms upon TLA - and placebo treatment, respectively. 23% of the patients in this subgroup subjected placebo treatment experienced a worsening of activity limitations due to rhinitis symptoms whereas 0% of the patients subjected to TLA treatment experienced a worsening of said limitations.

In the study the TLA device delivered air at a temperature 0.5-1 °C cooler than the ambient air and at this setting there were no reports of patients experiencing drafts.

### Example 2: Treatment of asthma using a specific TLA device configuration

A warm human body lying in a bed causes a convection flow transporting a high concentration of allergens and airborne particles to the person's breathing zone. As shown in Figure 1, the warm body of a sleeping person generates such a convection air currents.

A TLA device such as the one illustrated in Figure 3 provides a descending stream of filtered air that has sufficient velocity to overcome these convection body currents, as shown in Figure 2. The air-temperature of the air delivered into the treated air zone is 0.5 to 1 °C cooler than the ambient air surrounding the treated air zone resulting in the displacement of warm body convection currents without exposing the patient to an unpleasant draft. The zone of treated air provided by such devices may provide more than 95% reduction in airborne fine particle counts. The generation of such a controlled personal breathing zone that is substantially free of in-mixed, contaminated ambient air allows for reduction or removal of symptoms of perennial allergic asthma without exposing the patient to an unpleasant draft.

### Example 3: Treatment of allergic rhinitis using a specific TLA device configuration

A warm human body lying in a bed causes a convection flow transporting a high concentration of allergens and airborne particles to the person's breathing zone. As shown in Figure 1, the warm body of a sleeping person generates such a convection air currents.

A TLA device such as the one illustrated in Figure 3 provides a descending stream of filtered air that has sufficient velocity to overcome these convection body currents, as shown in Figure 2. The air-temperature of the air delivered into the treated air zone is 0.5 to 1 °C cooler than the ambient air surrounding the treated air zone resulting in the displacement of warm body convection currents without exposing the patient to an unpleasant draft. The zone of treated air provided by such devices may provide more than 95% reduction in airborne fine particle counts. The generation of such a controlled personal breathing zone that is substantially free of in-mixed, contaminated ambient air allows for reduction or removal of symptoms of allergic rhinitis without exposing the patient to an unpleasant draft.

### Example 4: Improvement of quality of sleep using a specific TLA device configuration

A warm human body lying in a bed causes a convection flow transporting a high concentration of allergens and airborne particles to the person's breathing zone. As shown in Figure 1, the warm body of a sleeping person generates such a convection air currents.

A TLA device such as the one illustrated in Figure 3 provides a descending stream of filtered air that has sufficient velocity to overcome these convection body currents, as shown in Figure 2. The air-temperature of the air delivered into the treated air zone is 0.5 to 1 °C cooler than the ambient air surrounding the treated air zone resulting in the displacement of warm body convection currents without exposing the patient to an unpleasant draft. The zone of treated air provided by such devices may provide more than 95% reduction in airborne fine particle counts. When used during sleep the generation of such a controlled personal breathing zone that is substantially free of in-mixed, contaminated ambient air allows for improvement of quality of sleep without exposing the patient to an unpleasant draft.

### References

1. Masoli M, Fabian D, Holt S, Beasley R. The global burden of asthma: executive summary of the GINA Dissemination committee report. Allergy 2004; 59(5): 469-78.
2. Marion RJ, Creer TL, Reynolds RV. Direct and indirect costs associated with the management of childhood asthma. Ann Allergy. 1985 jan;54(1):31-4.
3. Accordini S, Bugiani M, Arossa W, Gerzeli S, Marinoni A, Olivieri M, Pirina P, Carrozzi L, Dallari R, De Togni A, de Marco R. Poor control increases the economic cost of asthma. A multicentre population-based study. Int Arch Allergy Immunol. 2006;141 (2):189-98. Epub 2006 Aug 4.
4. Levy ML, Fletcher M, Price DB, Hausen T, Halbert RJ, Yawn BP. International Primary Care Respiratory Group (IPCRG) Guidelines: diagnosis of respiratory diseases in primary care. Prim Care Respir J. 2006 Feb;15(1):20-34. Epub 2005 Dec 27.
5. Taylor DR, Bateman ED, Boulet LP, Boushey HA, Busse WW, Casale TB, Chanez P, Enright PL, Gibson PG, de Jongste JC, Kerstjens HA, Lazarus SC, Levy ML, O'Byrne PM, Partridge MR, Pavord ID, Sears MR, Sterk PJ, Stoloff SW, Szefler SJ, Sullivan SD, Thomas MD, Wenzel SE, Reddel HK. A new perspective on concepts of asthma severity and control. Eur Respir J. 2008 Sep;32(3):545-54.
6. Busse WW. Lemanske RF jr. Asthma. N Eng. J Med 2001, 344(5):350-62.
7. Tan WC, Tan CH, Teoh PC. The role of climatic conditions and histamine release in exercise-induced bronchoconstriction. Ann Acad Med Singapore. 1985 Jul;14(3):465-9.
8. ATS/ERS recommendations for standardized procedures for the online and offline measurement of exhaled lower respiratory nitric oxide and nasal nitric oxide, 2005. Am J Respir Crit Care Med 2005;171:912-930.
9. Ehrs PO, Nokela M, Stallberg B, Hjemdahl P, Wikström Jonsson E. Brief questionnaires for patient-reported outcomes in asthma: validation and usefulness in a primary care setting. Chest. 2006 Apr;129(4):925-32.
10. Juniper EF, Guyatt GH, Cox FM, Ferrie PJ, King DR. Development and validation of the Mini Asthma Quality of Life Questionnaire. Eur Respir J. 1999 Jul;14(1):32-8.
11.Juniper EF, Buist AS, Cox FM, Ferrie PJ, King DR. Validation of a standardised version of the Asthma Quality of Life Questionnaire. Chest 1999; 115:1265-1270.
12. Juniper EF, Guyatt GH, Feeny DH, Ferrie PJ, Griffith LE, Townsend M. Measuring quality of life in children with asthma. Qual Life Res 1996; 5: 35-46.
13. Raat H, Bueving HJ, de Jongste JC, Grol MH, Juniper EF, van der Wouden JC. Responsiveness, Longitudinal and cross-sectional construct validity of the Paediatric Asthma Quality of Life Questionnaire (PAQLQ) in a Dutch population of children with asthma. Quality of Life Research 2005; 14: 265-72.
14. Custovic A, Simpson A, Woodcock A. Importance of indoor allergens in the induction of allergy and elicitation of allergic disease. Allergy. 1998;53(48 Suppl):115-20. Review.
15. Piacentini DG, Piacentini GL, Costella S, Vicentini L, Peroni D, Zanolla L, Bones AL. Allergen avoidance is associated with a fall in exhaled nitric oxide in asthmatic children. J Allergy Clin Immunol. 1999 Dec;104(6):1323-4.
16. Peroni DG, Piacentini GL, Costella S, Pietrobelli A, Bodini A, Loiacono A, Aralla R, Boner AL. Mite avoidance can reduce air trapping and airway inflammation in allergic asthmatic children. Clin Exp Allergy. 2002 Jun;32(6):850-5.
17. Craig TJ, Ferguson BJ, Krouse JH. Sleep impairment in allergic rhinitis, rhinosinusitis, and nasal polyposis. Am J Otolaryngol. 2008 May-Jun;29(3):209-17)
18. Juniper EF, Guyatt GH, Griffith LE, Ferrie PJ. Interpretation of rhinoconjunctivitis quality of life questionnaire data. J Allergy Clin Immunol 1996; 98: 843-845
19. Clearing the Air: Asthma and Indoor Air exposure, Institute of Medicine (U.S.), 2000, p 367-378. ISBN 0-309-06496-1
20. Kilburn S, Lasserson TJ, McKean M. P et allergen control measures for allergic asthma in children and adults.Cochrane Database Syst Rev. 2003;(1):CD002989.
21. Sheikh A, Hurwitz B, Shehata Y. House dust mite avoidance measures for perennial allergic rhinitis. Cochrane Database Syst Rev. 2007;(1):CD001563.
22. GINA 2009: Global Initiative for Asthma; A Global Strategy for Asthma Management and Prevention (www.ginaasthma.org).
23. Pedroletti, C., Millinger, E., Dahlén, B., Söderman, P., Zetterström, O. Clinical effects of purified air administered to the breathing zone in allergic asthma: A double-blind randomized cross-over trial. Respir Med 2009 Sep;103(9):1313-9.

## Claims

1. An air-treatment device having at least one filter for reducing or removing the symptoms of asthma and/or rhinitis in a patient in need thereof by delivering treated air into a zone around the patient generating a treated air zone
**characterized in that**
- the device comprises:
i) a temperature adjustment device (4), and
ii) a temperature control system (5) comprising a first temperature sensor placed in the supply air channel after the temperature adjustment device (4), and a second sensor placed in such manner as to measure ambient air at the level of the personal breathing zone but outside the effective stream of supply air,
which maintain the treated air temperature such that the temperature of the air delivered into the treated air zone at the point of care is 0.5 to 1 ºC cooler than the ambient air surrounding the treated air zone
whereby
- treated air descends in a laminar fashion and
- warm body convection currents are displaced without the patient being exposed to unpleasant draft.

2. A device according to claim 1 wherein the temperature of the air delivered into the treated air zone is 0.6-0.8 °C cooler than the ambient air surrounding the treated air zone.

## Patentansprüche

1. Eine Luftbehandlungsvorrichtung umfassend wenigstens einen Filter für Minderung oder Entfernung von Asthma- und/oder Rhinitis-Symptomen in einem bedürftigen Patienten durch Zufuhr von behandelter Luft in eine Zone um den Patienten herum, wodurch eine Zone von behandelter Luft entsteht, **dadurch gekennzeichnet, dass**
- die Vorrichtung:
i) eine Temperaturregulierungsvorrichtung (4), und
ii) ein Temperatursteuerungssystem (5) umfassend einen ersten Temperatursensor, der in dem Zufuhrluftkanal nach der Temperaturregulierungsvorrichtung (4) angeordnet ist, und einen zweiten Sensor, der so angeordnet ist, dass er die Umgebungsluft auf der Ebene der persönlichen Atmungszone aber ausserhalb des effektiven Zufuhrluftstroms messen kann,
umfasst,
womit das Temperatur der behandelten Luft aufrechterhalten wird auf so einer Weise dass das Temperatur der Luft, die in die Zone von behandelter Luft zugeführt wird, auf der Einsatzstelle 0,5 - 1 °C kälter ist als die Umgebungsluft, die die Zone von behandelter Luft umgibt,
wodurch
- behandelte Luft auf eine laminare Weise hinuntersteigt und
- warme Strömungen von Körperkonvektion abgebremst werden ohne dem Patienten unangenehme Zugluft herbeizuführen.

2. Eine Vorrichtung nach Anspruch 1, wobei das Temperatur der Luft, die in die Zone von behandelter Luft zugeführt wird, 0,6 - 0,8 °C kälter ist als die Umgebungsluft, die die Zone von behandelter Luft umgibt.

## Revendications

1. Dispositif de traitement de flux d'air ayant au moins un filtre pour réduire ou éliminer les symptômes d'asthme et/ou de rhinite chez un patient qui en a besoin en fournissant de l'air traité dans une zone autour du patient générant une zone d'air traitée
caractérisé en ce
- le dispositif comprend:
i) un dispositif d'ajustement de température (4), et
ii) un système de contrôle de température (5) comprenant un premier capteur de température placé dans le canal d'alimentation d'air après le dispositif d'ajustement de température (4), et un deuxième capteur placé de manière à pouvoir mesurer l'air ambiant au niveau de la zone de respiration personnelle, mais en dehors du jet efficace de l'alimentation d'air,
qui maintiennent la température de l'air traité de manière à ce que la température de l'air fourni dans la zone d'air traité au point des soins est de 0.5 à 1°C plus froide que l'air ambiant qui entoure la zone d'air traité
par quel moyen
- de l'air traité descend de manière laminaire et
- des courants de convection du corps chauds sont déplacés sans que le patient est exposé à un courant d'air désagréable.

2. Dispositif selon la revendication 1 dans lequel la température de l'air fourni dans la zone traitée est de 0.6 à 0.8°C plus froide que l'air ambiant qui entoure la zone de l'air traitée.
